# EUROPÄISCHE PATENTANMELDUNG

(11) **EP 0 733 377 A2**
(43) Veröffentlichungstag der Anmeldung: **25.09.1996**
(21) Anmeldenummer: 96103073.1
(22) Anmeldetag: 29.02.1996
(51) Int. Cl.: A61M 1/02

(54) **Blutentnahmegerät**

(30) Priorität: 23.03.1995 DE 29504897 U
(71) Anmelder: TRANSMED Medizintechnik GmbH, D-33181 Wünnenberg (DE)
(72) Erfinder: Rappert, Klaus-Jürgen, D-33181 Wünnenberg (DE); Alt, Erwin, D - 33181 Wünnenberg (DE)
(74) Vertreter: Kehl, Günther, Dipl.-Phys.

(57) **Zusammenfassung**

Blutentnahmegerät (1) mit einer Aufnahmeschale (2) zur Aufnahme eines Blutbeutels, mit einer durch ein Mikroprozessorsystem gesteuerten Wiegeeinrichtung zur laufenden Bestimmung des Gewichts des Blutbeutels und mit einer an die Wiegeeinrichtung angeschlossenen elektronischen Auswertungsschaltung. Um ein Blutentnahmegerät zu schaffen, bei dem die während der Blutentnahme ermittelten Daten, wie Blutmenge, Entnahmedauer etc. auf bequeme Weise und verwechslungssicher auf einen Rechner übertragen werden können, sind ein Einführschacht (3) und Anschlüsse für eine in den Einführschacht (3) einführbare elektronische Speicherkarte (4) vorgesehen.

## Beschreibung

Die Erfindung betrifft ein Blutentnahmegerät mit einer Aufnahmeschale zur Aufnahme eines Blutbeutels, mit einer durch ein Mikroprozessorsystem gesteuerten Wiegeeinrichtung zur laufenden Bestimmung des Gewichts des Blutbeutels und mit einer an die Wiegeeinrichtung angeschlossenen elektronischen Auswerteschaltung.

Bei der Blutentnahme wurden bisher Daten per Hand oder maschinell auf die Etiketten der Blutbeutel aufgebracht. Die während der Entnahme ermittelten Daten, wie Blutmenge, Entnahmedauer und Alarme mußten anschließend handschriftlich in den Begleitpapieren vermerkt werden. Dabei sind Fehler, insbesondere Verwechslungsmöglichkeiten nicht auszuschließen.

Es ist auch schon bekannt geworden, Blutentnahmegeräte der eingangs genannten Art direkt an einen Rechner anzukoppeln und die bei der Blutentnahme erfaßten Daten über diese Ankopplung direkt auf den Rechner zu übertragen. Bei dauerhaft eingerichteten Blutentnahmestationen ist dies eine zufriedenstellende Lösung. Häufig werden jedoch Blutentnahmestationen ambulant in Schulgebäuden oder Turnhallen eingerichtet und in solchen Fällen ist es sehr aufwendig, die Blutentnahmegeräte mit dem Rechner zu verbinden. In der Praxis mußten hierzu die Blutentnahmegeräte zu dem Rechner transportiert und die Daten auf diesen übertragen werden. Dies ist sehr zeitaufwendig und für die Blutentnahmegeräte besteht die Gefahr, daß sie während des Transportes beschädigt werden.

Der Erfindung liegt die Aufgabe zugrunde, ein Blutentnahmegerät der eingangs genannten Art zu schaffen, bei dem die während der Blutentnahme ermittelten Daten, wie Blutmenge, Entnahmedauer und Alarme auf bequeme Weise und verwechslungssicher auf einen Rechner übertragen werden können. Das Blutentnahmegerät soll darüber hinaus kostengünstig herzustellen sein.

Diese Aufgabe ist dadurch gelöst, daß das Blutentnahmegerät einen Einführschacht und Anschlüsse für eine in den Einführschacht einführbare elektronische Speicherkarte aufweist.

Auf diese Weise kann der Datenaustausch zwischen dem Blutentnahmegerät und einer externen Datenverarbeitungsanlage mittels eines leicht entnehmbaren Datenträgers in Scheckkartengröße erfolgen. Die Übertragung der Daten ist zuverlässig und die Übergabe der Speicherkarte, die die Größe einer normalen Scheckkarte haben kann, erfordert keinen besonderen Aufwand. Das Blutentnahmegerät ist kostengünstig herzustellen, da es keine teueren Laufwerke zur Speicherung der Daten über Festplatten oder Disketten enthalten muß. Auch das eventuelle Verlegen von Kabeln zwischen dem Blutentnahmegerät und einer externen Datenverarbeitungsanlage entfällt. Die Speicherkarten können gesammelt und nach Durchführung der Blutspendeaktion zur Datenverarbeitungsanlage gebracht werden.

Nach einer vorteilhaften Ausgestaltung der Erfindung enthält die Speicherkarte einen statischen Speicher (S-RAM) und mindestens eine Batterie. Bei netzunabhängigem Betrieb der Waage ergibt sich infolge der im Vergleich zu Festplatten- oder Diskettenlaufwerken geringen Stromaufnahme der Speicherkarte eine hohe Betriebsdauer.

Alternativ oder zusätzlich hierzu kann die Speicherkarte einen elektrisch programmierbaren oder löschbaren Festwertspeicher (EEPROM oder Flash-EPROM) enthalten.

Nach einer weiteren vorteilhaften Ausführungsform der Erfindung enthält das Blutentnahmegerät eine elektrisch betätigbare durch das Mikroprozessorsystem gesteuerte Schlauchklemme, über die der Verbindungsschlauch zwischen dem Blutbeutel und dem Patienten abgeklemmt werden kann. Auf diese Weise kann die Blutentnahme bei Erreichen der vorgesehenen Spendemenge automatisch präzise gestoppt werden, auch dann, wenn das Betreuungspersonal durch einen anderen Patienten momentan in Anspruch genommen wird.

Nach einer weiteren vorteilhaften Ausführungsform der Erfindung ist die Aufnahmeschale mit einem Antrieb versehen, der die Aufnahmeschale zeitweise in eine Schwenkbewegung versetzt. Dadurch wird während der Entnahme das Blut kontinuierlich mit dem in dem Blutbeutel enthaltenen Stabilisator vermischt.

Nach einer weiteren vorteilhaften Ausführungsform der Erfindung enthält das Blutentnahmegerät Mittel zur Bestimmung der Blutdurchflußmenge. Diese können beispielsweise aus einer Rechenschaltung bestehen, die aufgrund des laufenden Gewichts des Blutbeutels und der Zeit die Blutdurchflussmenge bestimmt. Mit einer Einheit zur Anzeige einer Blutdurchflußstörung kann beispielsweise angezeigt werden, wenn die Blutdurchflußmenge einen bestimmten vorgegebenen Wert unterschreitet.

Die Erfindung wird im folgenden an Hand des in den Figuren dargestellten Ausführungsbeispieles näher erläutert. Es zeigt:
- Figur 1.: Ein Blutentnahmegerät gemäß der Erfindung.
- Figur 2:: Eine Speicherkarte.

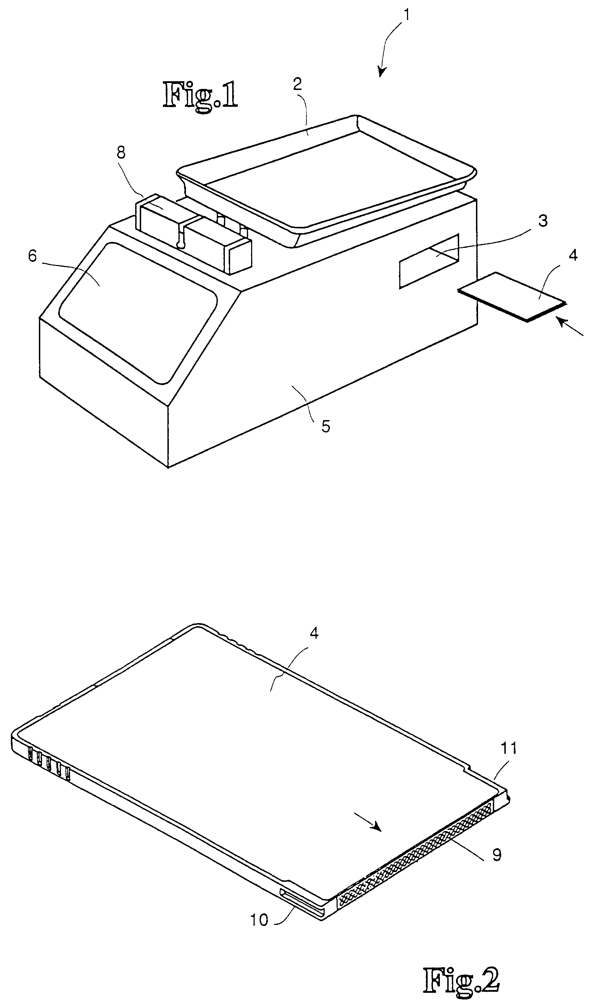

Figur 1 zeigt das Blutentnahmegerät gemäß der Erfindung in perspektivischer Darstellung. Das Blutentnahmegerät 1 besteht aus einem etwa quaderförmigen Gehäuse 5, das mit einem Tastenfeld 6 versehen ist. An der oberen Seite des Gehäuses befindet sich eine Schale 2, auf die ein Blutbeutel (nicht dargestellt) gelegt werden kann. Die Schale 2 ist mit einer innerhalb des Gehäuses angeordneten elektronischen Wiegeeinrichtung (nicht dargestellt) verbunden. Auf der oberen Fläche des Gehäuses ist des weiteren eine elektrisch betätigbare Schlauchklemme 8 angeordnet. Die Schlauchklemme ist mit dem im Inneren des Gehäuse angeordneten Mikroprozessorsystem (nicht dargestellt) verbunden, über das die Schlauchklemme bei Erreichen des vorgegebenen Gewichts geschlossen wird.

An einer Seitenwand des Blutentnahmegerätes ist ein Einführschacht 3 vorgesehen, an dessen innerem Ende (in der Zeichnung nicht zu erkennen) Steckkontakte angeordnet sind. Der Einführschacht kann auch an jeder anderen Wand des Geräts oder auch in einem über Kabel verbundenen, gesonderten Gehäuseteil angeordnet sein. Die Steckkontakte sind ebenfalls mit dem im Inneren des Gehäuses angeordneten Mikroprozessorsystem (nicht dargestellt) verbunden. In den Aufnahmeschacht 3 kann eine Speicherkarte 4 eingeführt werden, die zu den Anschlüssen im Inneren des Schachtes passende Steckkontakte aufweist, so daß die Speicherkarte 4 über diese Steckkontakte mit dem Mikroprozessorsystem verbunden werden kann.

Die Speicherkarte 4 ist in Figur 2 in perspektivischer Ansicht dargestellt. Die Speicherkarte 4, die etwa die Größe einer üblichen Scheckkarte aufweist, ist rechteckig und enthält seitliche Führungsnuten 10, 11, die ein präzises und seitenrichtiges Einsetzen der Speicherkarte 4 in das Blutentnahmegerät gewährleisten. In Figur 2 sind Anschlüsse 9 zu erkennen, mit denen die Speicherkarte mit dem Mikroprozessorsystem des Blutentnahmegerätes verbunden werden kann. Die Speicherkarte enthält einen statischen Speicher (S-RAM) sowie zwei Batterien. Durch das Vorsehen der zweiten Batterie oder eines Speicherkondensators kann ein Batteriewechsel ohne Datenverlust erfolgen, da die zweite Batterie oder der Speicherkondensator im Falle des Batteriewechsels die Aufrechterhaltung der Stromversorgung für das statische RAM gewährleistet.

Das Blutentnahmegerät gemäß der Erfindung arbeitet wie folgt: Auf die Aufnahmeschale 2 wird ein Blutbeutel gelegt, der die für die Konservierung des Blutes erforderlichen Stabilisatoren enthält. Der Blutentnahmeschlauch des Blutentnahmebeutels wird über die elektrische Schlauchklemme 8 zu dem Patienten geführt. Die Speicherkarte 4 wird in den Entnahmeschacht 3 eingeschoben, so daß sie mit dem Mikroprozessorsystem des Gerätes elektrisch verbunden wird.

Während der Blutentnahme wird die Aufnahmeschale zeitweise geschwenkt, so daß sich das in dem Beutel ansammelnde Blut mit dem dort befindlichen Stabilisator vermischt. Durch die Wiegeeinrichtung wird das Gewicht des Blutbeutels und indirekt die Durchflußmenge laufend erfaßt und bei Erreichen der vorgegebenen Blutmenge wird über das Mikroprozessorsystem die Schlauchklemme 8 geschlossen, wodurch die Blutentnahme automatisch beendet wird. Daten wie Blutmenge, Entnahmedauer und Alarme sowie extern erfaßte Daten, wie beispielsweise Dienstausweise, Spendernummer, Ortskennung der Entnahmestation werden in den Speicher der Speicherkarte 4 eingeschrieben und können nach Herausnehmen der Speicherkarte und Einführen der Speicherkarte in den Aufnahmeschacht einer externen Datenverarbeitungsanlage auf diese übertragen werden.

## Patentansprüche

1. Blutentnahmegerät (1) mit einer Aufnahmeschale (2) zur Aufnahme eines Blutbeutels, mit einer durch ein Mikroprozessorsystem gesteuerten Wiegeeinrichtung zur laufenden Bestimmung des Gewichts des Blutbeutels und mit einer an die Wiegeeinrichtung angeschlossenen elektronischen Auswertungsschaltung, dadurch **gekennzeichnet**, daß ein Einführschacht (3) und Anschlüsse für eine in den Einführschacht (3) einführbare elektronische Speicherkarte (4) vorgesehen sind.

2. Blutentnahmegerät (1) nach Anspruch 1, dadurch gekennzeichnet, daß die Speicherkarte (4) einen statischen Speicher (S-RAM) und mindestens eine Batterie enthält.

3. Blutentnahmegerät (1) nach Anspruch 1 oder 2, dadurch gekennzeichnet, daß die Speicherkarte (4) einen elektrisch programmierbaren oder löschbaren Festwertspeicher (EEPROM oder Flash-EPROM) enthält.

4. Blutentnahmegerät (1) nach einem der Ansprüche 1 bis 3, dadurch gekennzeichnet, daß es eine elektrisch betätigbare, durch das Mikroprozessorsystem gesteuerte Schlauchklemme (8) aufweist, durch die der Verbindungsschlauch zwischen dem Blutbeutel und dem Patienten abgeklemmt werden kann.

5. Blutentnahmegerät (1) nach einem der Ansprüche 1 bis 4, dadurch gekennzeichnet, daß die Aufnahmeschale (2) mit einem Antrieb versehen ist, der die Aufnahmeschale (2) zeitweise in eine Schwenkbewegung versetzt.

6. Blutentnahmegerät (1) nach einem der Ansprüche 1 bis 5, dadurch gekennzeichnet, daß es Mittel zur Bestimmung der Blutdurchflußmenge aufweist.
